(19) **Europäisches Patentamt
European Patent Office
Office européen des brevets**

(11) **EP 3 766 424 B1**

(12) **EUROPEAN PATENT SPECIFICATION**

(45) Date of publication and mention
of the grant of the patent:
**01.03.2023 Bulletin 2023/09**

(51) International Patent Classification (IPC):
**A61B 5/16** $^{(2006.01)}$ **A61B 5/11** $^{(2006.01)}$
**A61B 5/113** $^{(2006.01)}$ **A61B 5/00** $^{(2006.01)}$

(21) Application number: **19766979.9**

(22) Date of filing: **13.03.2019**

(52) Cooperative Patent Classification (CPC):
**A61B 5/4809; A61B 5/1036; A61B 5/113;
A61B 5/6891; A61B 5/6892; A61B 5/7242;
A61B 5/7246; A61B 5/7278;** A61B 2562/0252

(86) International application number:
**PCT/JP2019/010362**

(87) International publication number:
**WO 2019/177052 (19.09.2019 Gazette 2019/38)**

(54) **SLEEP/WAKE DETERMINATION SYSTEM**

SCHLAF/WACH-BESTIMMUNGSSYSTEM

SYSTÈME DE DÉTERMINATION DE SOMMEIL/VEILLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.03.2018 JP 2018046886**

(43) Date of publication of application:
**20.01.2021 Bulletin 2021/03**

(73) Proprietors:
• **Minebea Mitsumi Inc.
Kitasaku-gun, Nagano 3890293 (JP)**
• **National University Corporation Chiba University
Chiba-shi, Chiba 263-8522 (JP)**

(72) Inventors:
• **TODOROKI, Shinsuke
Kitasaku-gun, Nagano 389-0293 (JP)**
• **ISONO, Shiroh
Chiba-shi Chiba 260-8670 (JP)**
• **IIDA, Norihito
Kitasaku-gun, Nagano 389-0293 (JP)**

(74) Representative: **Novagraaf Group
Chemin de l'Echo 3
1213 Onex/ Geneva (CH)**

(56) References cited:
EP-A1- 2 074 945    WO-A2-2007/052108
JP-A- H06 327 652    JP-A- 2004 097 496
JP-A- 2011 160 852    JP-A- 2015 223 215
JP-A- 2017 064 350    JP-A- 2017 113 379
US-B2- 8 838 411

• BIJOY LAXMI KOLEY ET AL: "Selection of
features for detection of Obstructive Sleep Apnea
events", INDIA CONFERENCE (INDICON), 2012
ANNUAL IEEE, IEEE, 7 December 2012
(2012-12-07), pages 991-996, XP032316637, DOI:
10.1109/INDCON.2012.6420761 ISBN:
978-1-4673-2270-6

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a sleeping/waking determining system (sleep/wake determination system) for determining a sleeping or waking state (asleep/awake) of a (human) subject on the basis of detection value of load detector.

BACKGROUND ART

For the sites of medical treatment and caregiving service, it is proposed to determine a state of a subject on the basis of such a (body weight) load of the subject on a bed as detected by load detector(s). In particular, for example, it is proposed to determine whether the subject is in a sleeping state or in a waking state, that is, to determine the subject's sleeping/waking state on the basis of the detected load of the subject.

Patent Literature 1 discloses a sleeping determining apparatus provided to calculate a body motion number, that is the number of body motions, of a person on a bedding place (a bed), on the basis of a detection result of a load detector, and determine that a subject is in a sleeping state on the basis of the calculated number of body motions.

EP 2 074 945 A1 discloses a sleep evaluation device with a sensor unit configured to detect body movements of a human subject on bedding, wherein the device is configured to determine whether the subject is in a sleeping state or in a waking state based on a time-averaged value of a standard deviation of body movement data obtained from the sensor unit.

**Citation List**

Patent Literature 1: Japanese Patent Application Laid-open No. 2016-123810

SUMMARY

**Technical Problem**

An object of the present invention is to provide a sleeping/waking determining system capable of performing a sleeping/waking determination for a subject with higher precision.

**Solution to the Problem**

The present invention provides a sleeping/waking determining system according to claim 1 and a bed system according to claim 5.

Examples thereof are detailed in the dependent claims.

According to the sleeping/waking determining system of the present invention, it is possible to perform a sleeping/waking determination for a subject with higher precision.

BRIEF DESCRIPTION OF THE DRAWINGS

Fig. 1 is a block diagram depicting a configuration of a sleeping/waking determining system according to an embodiment of the present invention.
Fig. 2 is an illustrative view depicting an arrangement of load detectors for a bed.
Fig. 3 is a flow chart depicting a method of sleeping/waking determination by using the sleeping/waking determining system.
Fig. 4 is a schematic graph depicting an aspect of variation of load values detected by the load detectors in both a resting period when a subject is only respiring and a period when the subject is performing a body motion.
Fig. 5(a) is an illustrative view conceptually depicting an aspect where the center of gravity of the subject oscillates or vibrates in a body axis direction of the subject according to the respirations of the subject.
Fig. 5(b) is a graph depicting an example of a respiratory waveform drawn on the basis of the oscillation of the center of gravity of the subject according to the respirations of the subject.
Figs. 6(a), 6(b), 6(c), 6(d), and 6(e) are graphs respectively depicting a relationship between a body motion of the subject and a consequent increase of an activity index, wherein Fig. 6(a) is a graph where the sleeping subject performs a turn-over, Fig. 6(b) is a graph where the sleeping subject performs a twitch, Fig. 6(c) is a graph where the sleeping subject moves his/her right hand, Fig. 6(d) is a graph where the waking subject is reading a book, and Fig. 6(e) is a graph where the waking subject is having a meal.

Fig. 7 is a block diagram depicting an overall configuration of a bed system according to a modified embodiment.

DESCRIPTION OF EMBODIMENT

<Embodiment>

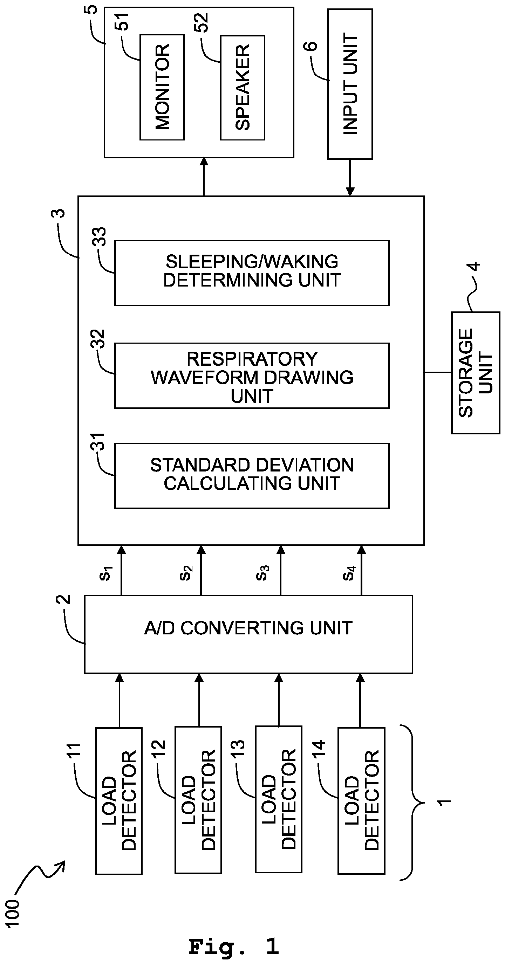

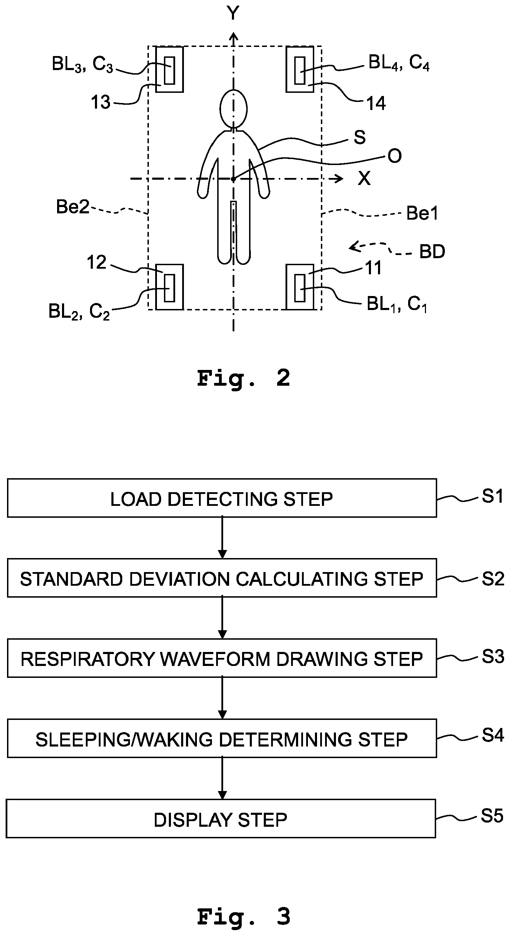

Explanations will be made on a sleeping/waking determining system 100 according to an embodiment of the present invention (Fig. 1), with an example of using the system together with a bed BD (Fig. 2) to determine whether a subject S on the bed BD is in a sleeping state or in a waking state.

As depicted in Fig. 1, the sleeping/waking determining system 100 of this embodiment primarily has a load detecting unit 1, a control unit 3, and a storage unit 4. The load detecting unit 1 and the control unit 3 are connected via an A/D converting unit 2. The control unit 3 is further connected to a display unit 5, and an input unit 6.

The load detecting unit 1 includes four load detectors 11, 12, 13, and 14. Each of the load detectors 11, 12, 13, and 14 is a load detector for detecting a load by using, for example, a beam-type load cell. Such a load detector is disclosed, for example, in Japanese Patent No. 4829020 and Japanese Patent No. 4002905. Each of the load detectors 11, 12, 13, and 14 is connected to the A/D converting unit 2 by way of wiring or wirelessly.

As depicted in Fig. 2, the four load detectors 11 to 14 of the load detecting unit 1 are arranged respectively under casters $C_1$, $C_2$, $C_3$, and $C_4$ fitted on the lower ends of legs $BL_1$, $BL_2$, $BL_3$, and $BL_4$ at the four corners of the bed BD used by the subject S.

The A/D converting unit 2 includes an A/D convertor connected respectively to the load detecting unit 1 and the control unit 3 by way of wiring or wirelessly, to convert analog signals fed from the load detecting unit 1 to digital signals.

The control unit 3 is a dedicated or general-purpose computer inside which a standard deviation calculating unit 31, a respiratory waveform drawing unit 32 (respiratory waveform obtaining unit; respiratory waveform calculating unit), and a sleeping/waking determining unit 33 are constructed.

The storage unit 4 is a storage device for storing data used in the sleeping/waking determining system 100 and, for example, a hard disk (magnetic disk) can be used for that purpose.

The display unit 5 is a part for performing a predetermined display on the basis of the outputs from the control unit 3, including a monitor 51 such as a liquid crystal monitor or the like to perform the display with images (videos), and a speaker 52 to perform the display with sounds.

The input unit 6 is an interface for performing predetermined inputs for the control unit 3, which may be a keyboard and a mouse.

An explanation will be made on an operation of determining the sleeping/waking state of the subject on the bed by using the sleeping/waking determining system 100 of such kind.

As depicted in the flow chart of Fig. 3, the determining of the subject's sleeping/waking state by using the sleeping/waking determining system 100, includes a load detecting step S1 for detecting the load of the subject S, a standard deviation calculating step S2 for calculating a standard deviation showing the degree of variation of the detected load, a respiratory waveform drawing step S3 for drawing a respiratory waveform of the subject on the basis of the detected load, a sleeping/waking determining step S4 for determining the subject's sleeping/waking state by using the standard deviation found in the standard deviation calculating step S2, and the respiratory waveform drawn in the respiratory waveform drawing step, and a display step S5 for displaying the result of the sleeping/waking determination.

[The load detecting step]

In the load detecting step S1, the load detectors 11, 12, 13, and 14 are used to detect the load of the subject S on the bed BD. The load of the subject S on the bed BD is applied dispersively to the load detectors 11 to 14 arranged respectively under the legs $BL_1$ to $BL_4$ of the bed BD at the four corners. The load of the subject S is detected dispersively by the four load detectors.

Each of the load detectors 11 to 14 detects the load (or the variation of load), and outputs the result as an analog signal to the A/D converting unit 2. The A/D converting unit 2 converts the analog signal into a digital signal at a sampling period of 5 milliseconds, for example, and then outputs the digital signal (to be referred to below as "load signal") to the control unit 3. Hereinafter, the term "load signals $s_1$, $s_2$, $s_3$, and $s_4$" will be used to refer respectively to the load signals obtained in the A/D converting unit 2 by converting the analog signals outputted from the load detectors 11, 12, 13, and 14 into the digital format.

[The standard deviation calculating step]

In the standard deviation calculating step S2, the standard deviation calculating unit 31 calculates standard deviations (moving standard deviations) $\sigma_1$, $\sigma_2$, $\sigma_3$, and $\sigma_4$ of a sampling value included in a predetermined sampling period (time period)(5 seconds for example) for each of load signals $s_1$, $s_2$, $s_3$, and $s_4$. The calculation may be performed at any time (or continuously).

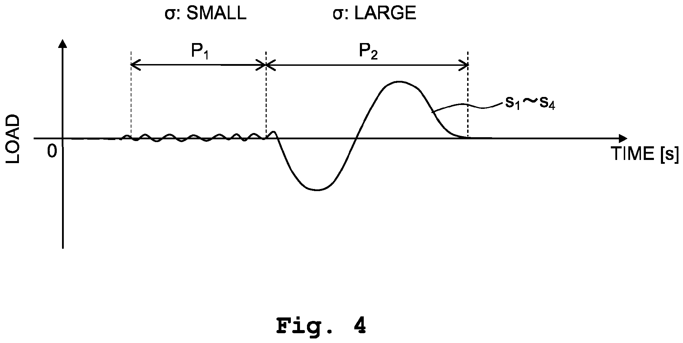

The standard deviation denotes the magnitude of variation (dispersion) of the sampling value. Thus, as depicted in Fig. 4, the standard deviations $\sigma_1$ to $\sigma_4$ become small during a period $P_1$ in which the subject S is resting on the bed BD and there is a small magnitude of variation in the load signals $s_1$ to $s_4$. On the other hand, the standard deviations $\sigma_1$ to $\sigma_4$ become large during a period $P_2$ in which the subject S is moving his/her body (in which there is a body motion arising in the subject S) and there is a large magnitude of variation in the load signals $s_1$ to $s_4$.

Therefore, during a period when there is a body motion arising in the subject S, the standard deviations $\sigma_1$ to $\sigma_4$ have larger values in comparison to a period when there is no body motion arising in the subject S (for example, a period when the subject only respires without moving his/her torso and/or hands and/or feet).

Note that in the present specification and in the present invention, the term "body motion" includes "large body motion" and "small body motion". The "body motion" does not include slight body movements of the subject due to the subject's respirations or heartbeats.

The large body motion refers to comparatively large ones of the subject's body motions accompanying a movement of his/her torso (trunk, body-trunk) such as, in particular for example, turn-over, sit-up or get-up, or the like). When a large body motion arises in the subject, generally speaking, there is a change in the orientation of the subject's body axis (the extending orientation of the subject's backbone).

The small body motion refers to comparatively small ones of the subject's body motions without accompanying a movement of his/her torso (trunk, body-trunk) such as, in particular for example, movements of only his/her hands, feet, head, or the like.

Generally speaking, the standard deviations $\sigma_1$ to $\sigma_4$ have larger values in the case where the subject S has a large body motion than in the case where the subject S has a small body motion.

[The respiratory waveform drawing step]

In the respiratory waveform drawing step S3, the respiratory waveform drawing unit 32 (respiratory waveform obtaining unit; respiratory waveform calculating unit) draws a respiratory waveform of the subject S on the basis of the load signals $s_1$ to $s_4$.

The respiration of human is performed by moving the chest and the diaphragm to expand and shrink the lungs. In this context, when the air is inhaled (or an inspiration is performed), i.e., when the lungs are expanded, the diaphragm is lowered downwardly, and the internal organs are also moved downwardly. On the other hand, when the air is expired (or an expiration is performed), i.e., when the lungs are shrunk, the diaphragm is raised upwardly, and the internal organs are also moved upwardly. As disclosed in the specification of Japanese Patent No. 6105703 granted to the present applicant, the center of gravity G (of the subject) moves slightly along with the movement of the internal organs, the moving direction of the center of gravity G being approximately along the subject's backbone extending direction (body axis direction).

In the present specification and in the present invention, the term "respiratory waveform" refers to a waveform showing an aspect of the oscillation of the subject's center of gravity oscillating in the subject's body axis direction due to the subject's respirations, by plotting the aspect on the temporal axis. One period of the respiratory waveform corresponds to one respiration of the subject (one inspiration and expiration). The amplitude of the respiratory waveform is affected by the subject's (physical) frame (build, physique) and/or respiratory depth. In particular, for example, if the subject has a large frame or the subject performs a deep respiration, then the amplitude becomes large, whereas if the subject has a small frame or the subject performs a shallow respiration, then the amplitude becomes small.

The respiratory waveform drawing unit 32 draws, in particular, the respiratory waveform in the following manner.

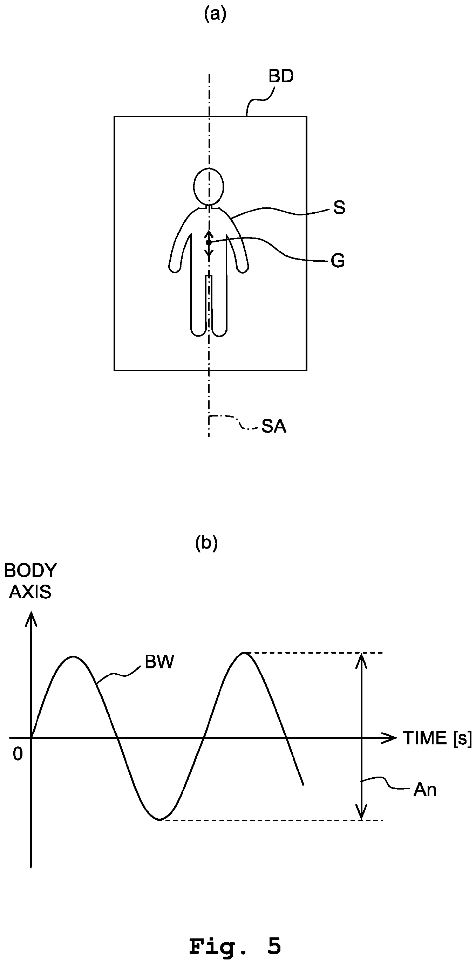

First, the respiratory waveform drawing unit 32 calculates the position of the center of gravity G of the subject S at each sampling time on the basis of the load signals $s_1$ to $s_4$ fed from the load detecting unit 1. As depicted in Fig. 5(a), the center of gravity G of the subject S oscillates in the direction of the body axis SA of the subject S in accordance with or due to the respiration of the subject S.

Next, the respiratory waveform drawing unit 32 draws a respiratory waveform BW (Fig. 5(b)) by way of plotting, on the vertical axis of a graph, the distance between the positions obtained by projecting the position of the center of gravity G at each time on the body axis SA, and the oscillation center of oscillation of the center of gravity G according to the respiration. The direction of the vertical axis of the graph matches the direction of the body axis SA. The horizontal axis of the graph shows time.

Note that it is not necessary for the respiratory waveform drawing unit 32 to actually draw the respiratory waveform but it is possible to only obtain data indicating the respiratory waveform.

[The sleeping/waking determining step]

In the sleeping/waking determining step S4, the sleeping/waking determining unit 33 uses the standard deviations $\sigma_1$ to $\sigma_4$ calculated in the standard deviation calculating step S2 and the amplitude of the respiratory waveform BW drawn in the respiratory waveform drawing step S3, to determine whether the subject S is in a sleeping state or in a waking state.

The determination is performed in the following manner in particular for example.

The sleeping/waking determining unit 33 first detect the peaks for the respiratory waveform BW drawn in the respiratory waveform drawing step S3 and, based on two successive peaks and the minimum value between those two peaks, obtains (finds) an amplitude An of the respiratory waveform BW (Fig. 5(b)). Then, the sleeping/waking determining unit 33 obtains a respiratory waveform average amplitude A by calculating a simple average of the amplitudes An found successively according to each period of the respiratory waveform BW.

Next, the sleeping/waking determining unit 33 obtains normalized standard deviations $\sigma s_1$ to $\sigma s_4$ by the following Formula 1.

[Formula 1]

$$\sigma s_n = \sigma_n / A \quad (n = 1, 2, 3, 4)$$

The reason for performing such a normalization is stated below.

As described earlier on, the standard deviations $\sigma_1$ to $\sigma_4$ have large values when the subject S has a body motion. In this context, since the standard deviations $\sigma_1$ to $\sigma_4$ show the magnitude of variation of the detection values of the load detectors 11 to 14 according to the body motion of the subject S, the increment or increase is larger for a subject S having a large frame than for a subject S having a small frame even if there is no difference in aspects of the body motion.

On the other hand, as described earlier on, the amplitude of the respiratory waveform is affected by the frame of the subject S; therefore, if the subject S has a large frame, then the amplitude An and the respiratory waveform average amplitude A become large, whereas if the subject S has a small frame, then the amplitude An and the respiratory waveform average amplitude A become small.

Therefore, as shown in the Formula 1, by dividing the values of the standard deviations $\sigma_1$ to $\sigma_4$ by the respiratory waveform average amplitude A to perform the normalization, it is possible to reduce (compensate) the influence on the values of the standard deviations $\sigma_1$ to $\sigma_4$, exerted by the subject's frame (physical description). Then, by using the normalized standard deviations $\sigma s_1$ to $\sigma s_4$ which are compensated in this manner, to perform the sleeping/waking determination for the subject S, it is possible to raise the precision of the determination.

Note that the normalization may be performed by dividing the values of the standard deviations $\sigma_1$ to $\sigma_4$ by any one of the amplitude An obtained right before and the amplitudes An obtained before, instead of the respiratory waveform average amplitude A.

Next, the sleeping/waking determining unit 33 calculates an activity index ACI which is a value obtained by performing a time-integration (temporal integration) of a simple average of the normalized standard deviations $\sigma s_1$ to $\sigma s_4$, by the following Formula 2.

[Formula 2]

$$ACI = \int_0^{20} \left( \frac{\sigma s_1 + \sigma s_2 + \sigma s_3 + \sigma s_4}{4} \right) dt$$

The integral time here in the formula is 20 seconds but, as will be described earlier on, it is not limited thereto. Because the normalized standard deviations $\sigma s_1$ to $\sigma s_4$ increase according to the subject's body motion, the activity index ACI becomes larger when the subject S shows a body motion bringing about a larger load variation over a longer time. That is, the activity index ACI is a parameter reflecting both the magnitude of the body motion and the continuance time (duration time) of the body motion.

Note that the following is the reason for obtaining the simple average of the normalized standard deviations

$\sigma s_1$ to $\sigma s_4$ in the Formula 2. That is, the balance of the values of the normalized standard deviations $\sigma s_1$ to $\sigma s_4$ varies according to the position of the subject S on the bed BD and, for example, if the center of gravity G of the subject S is positioned in the vicinity of the load detector 11, then the value of the normalized standard deviation $\sigma s_1$ becomes larger than the value of another normalized standard deviation. In such a case, for example, the value of the normalized standard deviation $\sigma s_2$ may not increase sufficiently even though the subject S shows a large body motion. By obtaining the simple average of the normalized standard deviations $\sigma s_1$ to $\sigma s_4$, it is possible to suppress such influence of the position of the subject S on the bed BD.

The sleeping/waking determining unit 33 calculates a new activity index ACI every 20 seconds, by using the values of the normalized standard deviations $\sigma s_1$ to $\sigma s_4$ at each sampling time over the past 20 seconds. Then, the sleeping/waking determining unit 33 determines whether the subject S is in a sleeping state or in a waking state on the basis of a comparison between the calculated activity index ACI and a predetermined threshold value.

The comparison between the activity index ACI and the threshold value is performed in the following manner, for example.

As a first example, if any one of the latest four values of the activity indexes ACI calculated every 20 seconds becomes larger than the predetermined threshold value, then the subject S is determined as in a waking state. As a second example, if the total of the latest three values of the activity indexes ACI calculated every 20 seconds becomes larger than the predetermined threshold value, then the subject S is determined as in a waking state. In this manner, it is possible to further raise the precision of the determination by performing determination using not only the latest activity index ACI but also a plurality of activity indexes ACI obtained over a certain length of time.

Hereinbelow, an explanation will be made on the reason why it is possible to raise the precision of the sleeping/waking determination for the subject S by using the activity index ACI.

Generally speaking, a person shows a smaller amount of body motion during a sleeping period than during a waking period. However, he or she may still present turn-overs, or slight motions referred to as "twitch" (spasm motion of muscle considered to occur in REM sleep). Further, he or she may move his or her hands, feet, and/or head to change the sleeping posture. Therefore, even by determining the presence or absence of body motion or the number of body motions of the subject S from the values of the normalized standard deviations $\sigma s_1$ to $\sigma s_4$ and performing the sleeping/waking determination on the basis of the present or absence of body motion or the number of body motions, the precision of the determination still cannot be regarded as sufficient.

On the other hand, the activity index ACI is a value obtained by performing a time-integration of the simple average of the normalized standard deviations $\sigma s_1$ to $\sigma s_4$, which is the product of the magnitude of body motion (the magnitude of increase of the normalized standard deviations $\sigma s_1$ to $\sigma s_4$) and the continuance time of body motion (the length of the period of increase of the normalized standard deviations $\sigma s_1$ to $\sigma s_4$).

Therefore, even as the subject S shows a comparative large body motion in a period of 20 seconds, if the continuance time of the body motion is short, then the activity index ACI will not become so large in value. On the other hand, even as the subject S does not show a large body motion in a period of 20 seconds, if the subject S shows a small body motion continuously, then the activity index ACI will become large in value.

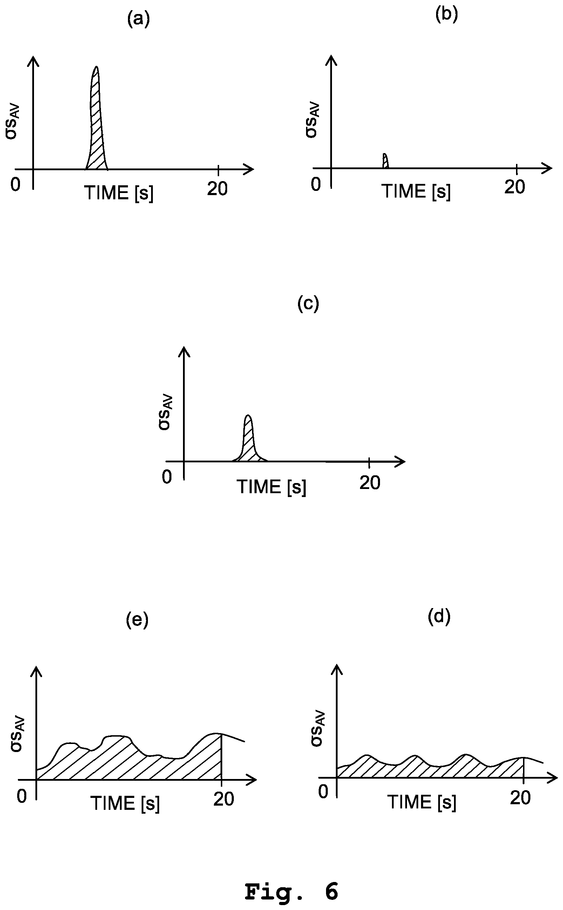

Figs. 6(a) to 6(e) schematically depict several specific examples.

Fig. 6(a) is a schematic graph depicting an aspect of variation of a simple average of the normalized standard deviations $\sigma s_1$ to $\sigma s_4$ (to be referred to below as $\sigma s_{AV}$) when the sleeping subject S performs a turn-over within a period of 20 seconds. The value of the activity index ACI according to this period corresponds to the area of the shaded part of the graph (much the same is true on Figs. 6(b) to 6 (e)).

Fig. 6(b) is a schematic graph depicting an aspect of variation of the simple average $\sigma s_{Av}$ when the sleeping subject S shows a twitch within a period of 20 seconds, and Fig. 6(c) is a schematic graph depicting an aspect of variation of the simple average $\sigma s_{AV}$ when the sleeping subject S shows a small body motion where the right hand changes from a flexed state to an extended state within a period of 20 seconds.

Fig. 6(d) is a schematic graph depicting an aspect of variation of the simple average $\sigma s_{AV}$ when the waking subject S reads a book within a period of 20 seconds, and Fig. 6(e) is a schematic graph depicting an aspect of variation of the simple average $\sigma s_{AV}$ when the waking subject S is having a meal within a period of 20 seconds.

As exhibited by Figs. 6(a) to 6(e), the value of the activity index ACI is inclined to have a larger value when the waking subject S shows a continuous body motion. Therefore, by properly setting a threshold value used in the determination, it is possible to perform the sleeping/waking determination with high precision by reducing the influence on the body motion determination, exerted by an instant body motion shown by the sleeping subject S such as a turn-over, a twitch, change in sleeping posture, or the like.

[The display step]

In the display step S5, the display unit 5 displays the determination result outputted from the control unit. In particular, for example, whether the subject S is in a sleeping state or in a waking state is constantly displayed on the monitor 51 and, meanwhile, if the subject S has transited from a sleeping state into a waking state, then the speaker 52 is used to notify the users of the transition auditorily.

The effects of the sleeping/waking determining system 100 of this embodiment are summarized as follows.

The sleeping/waking determining system 100 of this embodiment uses the activity index ACI to perform the sleeping/waking determination for the subject S. Therefore, there is suppressed influence on the determination, exerted by a body motion arising in the sleeping subject S over a short continuance time, such as a turn-over, a twitch, or the like, thereby providing the determination with high precision.

The sleeping/waking determining system 100 of this embodiment calculates the activity index ACI by using the normalized standard deviations $\sigma s_1$ to $\sigma s_4$ resulted from normalizing the values of the standard deviations $\sigma_1$ to $\sigma_4$ by the respiratory waveform average amplitude A. Therefore, there is reduced influence on the values of the standard deviations $\sigma_1$ to $\sigma_4$ and, furthermore, on the sleeping/waking determination, exerted by the physical description of the subject S, thereby providing the determination with high precision.

The body motion determining system 100 of this embodiment uses the load detectors 11 to 14 arranged under the legs $BL_1$ to $BL_4$ of the bed BD to determine a biological state of the subject S. Therefore, it is not necessary to attach any measuring device to the body of the subject S so that the subject S will not feel discomfort and a sense of incongruity.

[Modified embodiments]

It is also possible to adopt the following modified embodiments with respect to the sleeping/waking determining system 100 according to the above embodiment.

In the sleeping/waking determining system 100 of the above embodiment, the sleeping/waking determining unit 33 uses the Formula 2 to calculate the activity index ACI. However, the method for calculating the activity index ACI is not limited to that.

For example, the sleeping/waking determining unit 33 may find the activity index ACI by using the following Formula 3 where the normalized standard deviations $\sigma s_1$ to $\sigma s_4$ in the Formula 2 are replaced with the standard deviations $\sigma_1$ to $\sigma_4$ (not covered by the claimed invention).

[Formula 3]

$$\text{ACI} = \int_0^{20} \left( \frac{\sigma_1 + \sigma_2 + \sigma_3 + \sigma_4}{4} \right) dt$$

The sleeping/waking determining unit 33 may use a value obtained by performing a time-integration of maximum normalized standard deviations $\sigma s_{MAX}$ (covered by the claimed invention) or a value obtained by performing a time-integration of maximum standard deviations $\sigma_{MAX}$ (not covered by the claimed invention), as the value of the activity index ACI. The maximum normalized standard deviations $\sigma s_{MAX}$ are a series of selected values prepared by selecting the largest value of the normalized standard deviations $\sigma s_1$ to $\sigma s_4$ at each sampling time, and the maximum standard deviations $\sigma_{MAX}$ are a series of selected values prepared by selecting the largest value of the standard deviations $\sigma_1$ to $\sigma_4$ at each sampling time.

The sleeping/waking determining unit 33 may use, as the value of the activity index ACI, a value obtained by performing a time-integration of at least one of the normalized standard deviations $\sigma s_1$ to $\sigma s_4$ (covered by the claimed invention) or at least one of the standard deviations $\sigma_1$ to $\sigma_4$ (not covered by the claimed invention), instead of a simple average of the normalized standard deviations $\sigma s_1$ to $\sigma s_4$ or a simple average of the standard deviations $\sigma_1$ to $\sigma_4$.

The activity indexes ACI according to those modified embodiments are also parameters reflecting both the magnitude of the body motion and the continuance time (duration time) of the body motion. It is possible to use any other parameter which is obtained by performing a time-integration of the standard deviation of the temporal variation of the load of the subject and which reflects both the magnitude of the body motion and the continuance time (duration time) of the body motion, as the activity index ACI.

Note that in the explanations of the sleeping/waking determining system 100 according to the above embodiment and modified embodiments, the integral time is 20 seconds for calculating the activity index ACI. However, without being limited to that, the integral time may be set arbitrarily but, if the integral time is too short, then there will be unclear distinction between a body motion having a short continuance time and a body motion having a long continuance time. Conversely, if the integral time is too long, then there will be a long interval between the points of time of performing the sleeping/waking determination (a period of performing the determination is lengthen), thereby making it difficult to have a well-timed or timely determination. The sleeping/waking determining unit 33 calculates a new activity index ACI each time the set integral time has elapsed.

Further, in calculating the activity index ACI, it is also possible to use a variance which is the squared standard deviation instead of the standard deviation (not covered by the claimed invention). In the present specification, the value obtained by performing a time-integration of the variance is also included in "the value obtained by performing a time-integration of the standard deviation".

The sleeping/waking determining unit 33 may provide the threshold value for the comparison with the activity index ACI with hysteresis. In particular, for example, with a first threshold value and a second threshold value larger than the first threshold value set in advance, under the condition that the subject S is determined as in a sleeping state, as far as the activity index ACI is smaller than the second threshold value, the subject S is not determined as in a waking state. On the other hand, under the condition that the subject S is determined as in a waking state, even if the activity index ACI is smaller than the second threshold value, the subject S is not determined as in a sleeping state, but is determined as in a sleeping state at the point of the activity index ACI having turned smaller than the first threshold value.

The sleeping/waking determining system 100 of the above embodiment does not need to include all of the load detectors 11 to 14 but may include only any one of the four. For example, if the system is not provided with four load detectors, then the number of the normalized standard deviations $\sigma s_n$ (n = 1, 2, 3, 4) included in the Formula 2 and the number of the standard deviations $\sigma_n$ (n = 1, 2, 3, 4) included in the Formula 3 may also be accordingly reduced in correspondence with the number of load detectors. Further, the maximum normalized standard deviations $\sigma s_{MAX}$ and the maximum standard deviations $\sigma_{MAX}$ may also be based on the normalized standard deviations $\sigma s_n$ and the standard deviations $\sigma_n$ obtained in correspondence with the number of load detectors.

Further, the load detectors 11 to 14 are not limited to load sensors using beam-type load cells but, for example, force sensors are also usable.

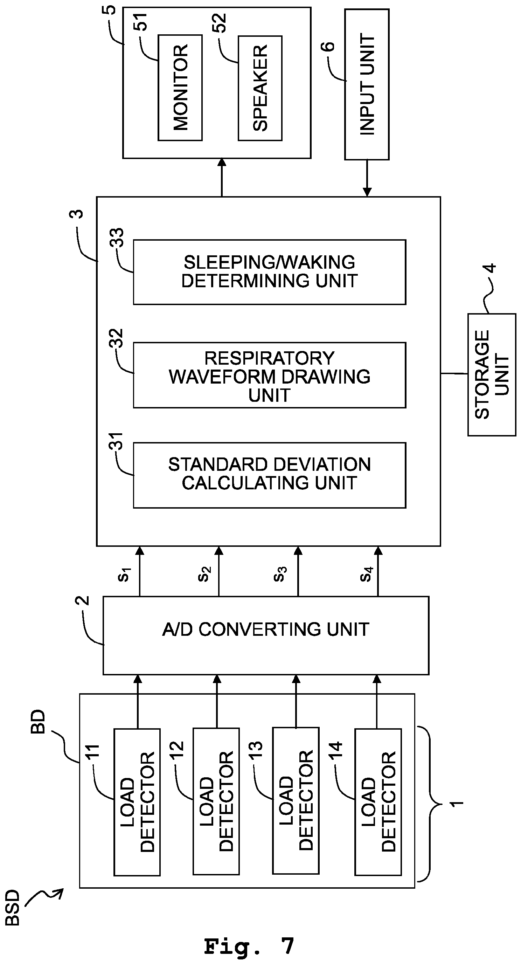

In the sleeping/waking determining system 100 of the above embodiment, the load detectors 11 to 14 are arranged respectively on the undersides of the casters C attached to the lower ends of the legs of the bed BD. However, there is no limitation thereto. Each of the load detectors 11 to 14 may be provided respectively between one of the four legs of the bed BD and the board of the bed BD. Alternatively, if each of the four legs of the bed BD can be divided into upper and lower portions, each of the load detectors 11 to 14 may be provided between the upper leg and the lower leg. Still alternatively, the load detectors 11 to 14 may be formed integral with or removable from the bed BD to construct a bed system BDS comprising the bed BD, and the sleeping/waking determining system 100 of the above embodiment (Fig. 7).

INDUSTRIAL APPLICABILITY

According to the sleeping/waking determining system of the present invention, it is possible to perform the sleeping/waking determination for the subject with high precision and, based on the high-precision determination, it is possible to provide high-quality medical treatments and caregiving services.

PARTS LIST

1: load detecting unit, 11, 12, 13, 14: load detector, 2: A/D converting unit, 3: control unit, 31: standard deviation calculating unit, 32: respiratory waveform drawing unit, 33: sleeping/waking determining unit, 4: storage unit, 5: display unit, 6: input unit, 100: sleeping/waking determining system, BD: bed, BDS: bed system, S: subject.

**Claims**

1. A sleeping/waking determining system (100) configured to determine whether a subject (S) on a bed (BD) is in a sleeping state or in a waking state, the system (100) comprising:

   a load detector (11-14) configured to detect a load of the subject (S) on the bed (BD);
   a determining unit (33) configured to determine whether the subject (S) is in the sleeping state or in the waking state by using a standard deviation of a temporal variation of the load of the subject (S); and
   a respiratory waveform obtaining unit (32) configured to obtain a respiratory waveform of the subject (S) based on the temporal variation of the load of the subject (S),
   **characterized in that**
   the determining unit (33) is configured to determine whether the subject (S) is in the sleeping state or in the waking state, based on a comparison between a threshold value and a value obtained by performing a time integration of a value which is obtained by dividing the standard deviation by an amplitude of the respiratory waveform.

**2.** The sleeping/waking determining system (100) according to claim 1, wherein the load detector (11-14) includes at least a first load detector (11) and a second load detector (12), and the standard deviation is a simple average of a first standard deviation of the temporal variation of the load of the subject (S) detected by the first load detector (11), and a second standard deviation of the temporal variation of the load of the subject (S) detected by the second load detector (12).

**3.** The sleeping/waking determining system (100) according to claim 2, wherein the load detector (11-14) further includes a third load detector (13) and a fourth load detector (14), and
the standard deviation is the simple average of the first standard deviation of the temporal variation of the load of the subject (S) detected by the first load detector (11), the second standard deviation of the temporal variation of the load of the subject (S) detected by the second load detector (12), a third standard deviation of the temporal variation of the load of the subject (S) detected by the third load detector (13), and a fourth standard deviation of the temporal variation of the load of the subject (S) detected by the fourth load detector (14).

**4.** The sleeping/waking determining system (100) according to claim 1, wherein the load detector (11-14) includes at least a first load detector (11) and a second load detector (12), and
the standard deviation is a series of values obtained by successively selecting the larger one of a first standard deviation and a second standard deviation at each time, the first standard deviation being a standard deviation of the temporal variation of the load of the subject (S) detected by the first load detector (11), the second standard deviation being a standard deviation of the temporal variation of the load of the subject (S) detected by the second load detector (12).

**5.** A bed system (BDS) comprising:

a bed (BD); and
the sleeping/waking determining system (100) as defined in any one of claims 1 to 4.


**Patentansprüche**

**1.** Schlaf-/Aufwachbestimmungssystem (100), das konfiguriert ist, um zu bestimmen, ob sich ein Subjekt (S) auf einem Bett (BD) in einem Schlafzustand oder in einem Aufwachzustand befindet, das System (100) umfassend:

einen Lastdetektor (11-14), der konfiguriert ist, um eine Last des Subjekts (S) auf dem Bett (BD) zu erfassen;
eine Bestimmungseinheit (33), die konfiguriert ist, um zu bestimmen, ob sich das Subjekt (S) in dem Schlafzustand oder in dem Aufwachzustand befindet, durch Verwenden einer Standardabweichung einer zeitlichen Variation der Last des Subjekts (S); und
eine Atemwellenformerhaltungseinheit (32), die konfiguriert ist, um eine Atemwellenform des Subjekts (S) basierend auf einer zeitlichen Variation der Last des Subjekts (S) zu erhalten,
**dadurch gekennzeichnet, dass**
die Bestimmungseinheit (33) konfiguriert ist, um zu bestimmen, ob sich das Subjekt (S) in dem Schlafzustand oder in dem Aufwachzustand befindet, basierend auf einem Vergleich zwischen einem Schwellenwert und einem Wert, der durch ein Durchführen einer Zeitintegration eines Werts erhalten wird, der durch ein Dividieren der Standardabweichung durch eine Amplitude der Atemwellenform erhalten wird.

**2.** Schlaf-/Aufwachbestimmungssystem (100) nach Anspruch 1, wobei der Lastdetektor (11-14) mindestens einen ersten Lastdetektor (11) und einen zweiten Lastdetektor (12) einschließt und die Standardabweichung ein einfacher Durchschnitt einer ersten Standardabweichung der zeitlichen Variation der Last des Subjekts (S), die durch den ersten Lastdetektor (11) erfasst wird, und einer zweiten Standardabweichung der zeitlichen Variation der Last des Subjekts (S) ist, die durch den zweiten Lastdetektor (12) erfasst wird.

**3.** Schlaf-/Aufwachbestimmungssystem (100) nach Anspruch 2, wobei der Lastdetektor (11-14) ferner einen dritten Lastdetektor (13) und einen vierten Lastdetektor (14) einschließt, und
die Standardabweichung der einfache Durchschnitt der ersten Standardabweichung der zeitlichen Variation der Last des Subjekts (S), die durch den ersten Lastdetektor (11) erfasst wird, der zweiten Standardabweichung der zeitlichen Variation der Last des Subjekts (S), die durch den zweiten Lastdetektor (12) erfasst wird, einer dritten Standardabweichung der zeitlichen Variation der Last des Subjekts (S), die durch den dritten Lastdetektor (13) erfasst wird, und einer vierten Standardabweichung der zeitlichen Variation der Last des Subjekts (S) ist, die durch den vierten Lastdetektor (14) erfasst wird.

**4.** Schlaf-/Aufwachbestimmungssystem (100) nach Anspruch 1, wobei der Lastdetektor (11-14) mindestens einen ersten Lastdetektor (11) und einen zweiten Lastdetektor (12) einschließt, und die Standardabweichung eine Reihe von Werten ist, die durch ein sukzessives Auswählen der größeren von einer ersten Standardabweichung und einer zweiten Standardabweichung zu jedem Zeitpunkt erhalten werden, wobei die erste Standardabweichung eine Standardabweichung der zeitlichen Variation der Last des Subjekts (S) ist, die durch den ersten Lastdetektor (11) erfasst wird, wobei die zweite Standardabweichung eine Standardabweichung der zeitlichen Variation der Last des Subjekts (S) ist, die durch den zweiten Lastdetektor (12) erfasst wird.

**5.** Bettsystem (BDS), umfassend:

ein Bett (BD); und
Schlaf-/Aufwachbestimmungssystem (100) nach einem der Ansprüche 1 bis 4.

**Revendications**

**1.** Système de détermination de veille/éveil (100) configuré pour déterminer si un sujet (S) sur un lit (BD) est dans un état de veille ou dans un état d'éveil, le système (100) comprenant :

un détecteur de charge (11-14) configuré pour détecter une charge du sujet (S) sur le lit (BD) ;
une unité de détermination (33) configurée pour déterminer si le sujet (S) est dans l'état de veille ou dans l'état d'éveil en utilisant un écart-type d'une variation temporelle de la charge du sujet (S) ; et
une unité d'obtention de forme d'onde respiratoire (32) configurée pour obtenir une forme d'onde respiratoire du sujet (S) en fonction de la variation temporelle de la charge du sujet (S),
**caractérisé en ce que**
l'unité de détermination (33) est configurée pour déterminer si le sujet (S) est dans l'état de veille ou dans l'état d'éveil, en fonction d'une comparaison entre une valeur seuil et une valeur obtenue en effectuant une intégration de temps d'une valeur qui est obtenue en divisant l'écart-type par une amplitude de la forme d'onde respiratoire.

**2.** Système de détermination de veille/éveil (100) selon la revendication 1, dans lequel le détecteur de charge (11-14) inclut au moins un premier détecteur de charge (11) et un deuxième détecteur de charge (12), et l'écart-type est une moyenne simple d'un premier écart-type de la variation temporelle de la charge du sujet (S) détectée par le premier détecteur de charge (11), et d'un deuxième écart-type de la variation temporelle de la charge du sujet (S) détectée par le deuxième détecteur de charge (12).

**3.** Système de détermination de veille/éveil (100) selon la revendication 2, dans lequel le détecteur de charge (11-14) inclut en outre un troisième détecteur de charge (13) et un quatrième détecteur de charge (14), et l'écart-type est la moyenne simple du premier écart-type de la variation temporelle de la charge du sujet (S) détectée par le premier détecteur de charge (11), le deuxième écart-type de la variation temporelle de la charge du sujet (S) détectée par le deuxième détecteur de charge (12), un troisième écart-type de la variation temporelle de la charge du sujet (S) détectée par le troisième détecteur de charge (13), et un quatrième écart-type de la variation temporelle de la charge du sujet (S) détectée par le quatrième détecteur de charge (14).

**4.** Système de détermination de veille/éveil (100) selon la revendication 1, dans lequel le détecteur de charge (11-14) inclut au moins un premier détecteur de charge (11) et un deuxième détecteur de charge (12), et l'écart-type est une série de valeurs obtenues en choisissant successivement le plus grand parmi un premier écart-type et un deuxième écart-type à chaque fois, le premier écart-type étant un écart-type de la variation temporelle de la charge du sujet (S) détectée par le premier détecteur de charge (11), le deuxième écart-type étant un écart-type de la variation temporelle de la charge du sujet (S) détectée par le deuxième détecteur de charge (12).

**5.** Système de lit (BDS) comprenant :

un lit (BD) ; et
le système de détermination de veille/éveil (100) tel que défini selon l'une quelconque des revendications 1 à 4.

Fig. 1

Fig. 2

Fig. 3

Fig. 4

(a)

(b)

**Fig. 5**

(a)

(b)

(c)

(e)

(d)

Fig. 6

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2074945 A1
- JP 2016123810 A
- JP 4829020 B
- JP 4002905 B
- JP 6105703 B